Europäisches Patentamt

**European Patent Office**

Office européen des brevets

(19)

(11) Veröffentlichungsnummer: **0 211 093**
**B1**

(12) # EUROPÄISCHE PATENTSCHRIFT

(45) Veröffentlichungstag der Patentschrift:
**20.09.89**

(51) Int. Cl.⁴: **A61K 35/78**

(21) Anmeldenummer: **85109616.4**

(22) Anmeldetag: **31.07.85**

(54) **Arzneimittel zur Behandlung von Herpes.**

(43) Veröffentlichungstag der Anmeldung:
**25.02.87 Patentblatt 87/9**

(45) Bekanntmachung des Hinweises auf die Patenterteilung:
**20.09.89 Patentblatt 89/38**

(84) Benannte Vertragsstaaten:
**AT BE CH DE FR GB IT LI LU NL SE**

(56) Entgegenhaltungen:
**EP-A- 0 019 808**

**BIOLOGICAL ABSTRACTS, Band 62, 1976, Nr. 22342,
Biological Abstracts Inc., Philadelphia, PA, US; R.J.
ROBINS et al.: "The nature of the stimuli causing
digestive juice secretion in Dionaea muscipula Ellis
(Venus's flytrap)", & PLANTA
(BERL) 128(3): 263-265, 1976 000**

(73) Patentinhaber: **Keller, Helmut, Dr.med.,
Blumenstrasse 32, D-8646 Nordhalben(DE)**

(72) Erfinder: **Keller, Helmut, Dr.med., Blumenstrasse 32,
D-8646 Nordhalben(DE)**

(74) Vertreter: **Freiherr von Pechmann, Eckehart et al,
Patentanwälte Wuesthoff- v. Pechmann-Behrens-Goetz
Schweigerstrasse 2, D-8000 München 90(DE)**

ACTORUM AG

## Beschreibung

Aus der EP 19 808 bzw. der entsprechenden Patentanmeldung 80 102 707 ist es bekannt, daß der Preßsaft bestimmter fleischfressender Pflanzen (Carnivoren) sich sur Krebsbekämpfung eignet. Als besonders wirksam haben sich die Inhaltsstoffe des Preßsaftes von Pflanzenteilen der Venusfliegenfalle (Dionaea muscipula) erwiesen. Diese Preßsäfte enthalten u.a. spezifische proteolytische Fermente und können oral oder nach Filtrieren und Verdünnen mit Kochsalzlösungen als Injektionspräparate appliziert werden. Wie in der Veröffentlichung: EHK Acta medica empirica, Bd. 34, 416-420 (1985) näher beschrieben ist, dürfte die Wirkung auch auf das Vorhandensein spezifischer Endonucleasen, die ebenfalls in den Preßsäften dieser fleischfressenden Pflanzen festgestellt wurden, zurückzuführen sein. Diese Wirkstoff bewirken eine Zellteilhemung bei den Tumorzellen, wodurch in vielen Fällen ein deutlicher Rückgang des Tumors bzw. sein Verschwinden erreicht werden konnte. Auch bei Patienten, die bereits Metastasen aufweisen, war die Therapie mit den im Handel unter der Bezeichnung CARNIVORA® der Firma Carnivora GmbH, Jagsthausen, erhältlichen Präparaten erfolgreich. Hierbei handelt es sich um verdünnte Preßsäfte oder um die lyophilisierten Feststoffe des Preßsaftes der frischen Pflanzenteile von Dionaea muscipula, die oral eingenommen oder nach Auflösen des lyophilisierten Pulvers in Wasser intramuskulär injiziert werden. Der Preßsaft kann auch nach Verdünnen mit Wasser in Form eines Aerosprays inhaliert werden.

Es wurde nun überraschenderweise festgestellt, daß der Preßsaft oder der wäßrige Extrakt von Teilen fleischfressender Pflanzen sich zur Behandlung der Herpes-Infektionen eignet und zwar sowohl bei oraler als auch bei lokaler Applikation. Als Herpes wird ein plötzlich auftretender Bläschenausschlag bezeichnet, der meist an den Übergangsstellen der Haut in die Schleimhäute auftritt, z.B. am Kopf im Lippenbereich oder an der Nase sowie an den Genitalien oder am After. Aber auch in anderen Bereichen des Körpers kann Herpes in Form des Herpes Zoster auftreten in Verbindung mit einer neuralgischen Affektion. Häufig tritt der Herpes infolge einer sonstigen Belastung des Organismus auf, z.B. bei fiebrigen Erkrankungen, starker Sonnenbestrahlung des Gesichts, usw. Die Bläschen können in schwer heilende Geschüre mit schmerzenden Schwellungen übergehen. Die bisherige Behandlungen der Herpes-Erkrankungen sind nicht befriedigend. Die Behandlung mit Tinkturen oder Zinksalben oder auch oral mit Vitamin B führt häufig nicht zu der gewünschten Besserung.

Erfindungsgemäß wird nun zur Herstellung eines Arzneimittels zur Behandlung von Herpes der Preßsaft oder wäßrige Extrakt von fleischfressenden Pflanzen verwendet. Das mit dem Saft oder Extrakt von Carnivoren-Pflanzen, insbesondere mit dem der Venusfliegenfalle (Dionaea muscipula), hergestellte Arzneimittel kann bei lokaler Applikation in flüssiger Form, gegebenenfalls nach Verdünnung mit Wasser und/oder verdünntem Alkohol mittels Kompressen oder durch Betupfen auf die erkrankten Hautbereiche aufgebracht werden. Schon nach einigen Tagen tritt eine deutliche Besserung der Entzündungen ein, die dann zu einer baldigen Abheilung führt. Wahrscheinlich werden durch die Inhaltsstoffe des Saftes dieser besonderen Pflanzen die erkrankten Hautge webe abgebaut und aufgelöst, so daß sich neue gesunde Haut bilden kann. Aber auch oral eingenommen bewirkt der Preßsaft oder Extrakt der Carnivoren-Pflanzen eine deutliche Besserung und nach einiger Zeit eine Abheilung der erkrankten Hautbereiche.

Um länger haltbare Präparate zu erhalten, kann der Preßsaft auch in üblicher Weise lyophilisiert werden. Nach Auflösung des Lyophilisats in Wasser oder verdünnter Kochsalzlösung wird dann die erhaltene Lösung lokal oder oral appliziert.

### Beispiel 1

Zur Herstellung des lokal applizierbaren Arzneimittels für die Behandlung von Herpes simplex werden die frisch geernteten Pflanzenteile von Dionaea muscipula in üblicher Weise ausgepreßt und der Saft gefiltert. Dieser kann unmittelbar auf die erkrankten Hautstellen aufgebracht werden oder es werden damit Kompressen imprägniert, die dann auf die Haut aufgelegt werden. Zur Stabilisierung können dem Preßsaft übliche Konservierungsmittel bzw. etwas Alkohol zugesetzt werden.

### Beispiel 2

Zur Herstellung eines oral anwendbaren Arzneimittels zur Behandlung der Herpes-Erkrankungen wird der gefilterte Preßsaft nach Beispiel 1 mit alkoholhaltigem Wasser oder Kochsalzlösung im Verhältnis von 1 : 3 verdünnt. Hiervon sollen mehrmals täglich ca. 2 ml (50 Tropfen) oral eingenommen werden.

### Patentansprüche

1. Verwendung des Preßsaftes oder wäßrigen Extrakes fleischfressender Pflanzen zur Herstellung eines Arzneimittels zur Behandlung von Herpes.

2. Verwendung des lyophilisierten Preßsaftes oder Extraktes der fleischfressenden Pflanzen zur Herstellung eines Arzneimittels zur Behandlung nach Anspruch 1.

3. Verwendung des Preßsaftes oder Extraktes oder des lyophilisierten Preßsaftes oder Extraktes der Pflanze Venusfliegenfalle (Dionaea muscipula) zur Herstellung eines Arzneimittels zur Behandlung nach Anspruch 1.

### Claims

1. Use of the pressed juice or aqueous extract of carnivorous plants for the production of a medicament for the treatment of herpes.

2. Use of the lyophilised pressed juice or extract of the carnivorous plants for the production of a medicament for the treatment according to claim 1.

3. Use of the pressed juice or extract or of the lyophilised pressed juice or extract of the plant Venus fly trap (Dionaea muscipula) for the production of a medicament for the treatment according to claim 1.

**Revendications**

1. Utilisation du jus de pression ou de l'extrait aqueux de plantes carnivores pour la fabrication d'un médicament pour le traitement de l'herpès.

2. Utilisation du jus de pression ou de l'extrait lyophilisé des plantes carnivores pour la fabrication d'un medicament pour le traitement selon la revendication 1.

3. Utilisation du jus de pression ou de l'extrait ou du jus de pression ou de l'extrait lyophilisé de la plante Dionée Gobe-Mouches (Dionaea muscipula) pour la fabrication d'un médicament pour le traitement selon la revendication 1.